(19)

Europäisches
Patentamt
European
Patent Office
Office européen
des brevets

(11) **EP 4 578 392 A1**

(12) **EUROPEAN PATENT APPLICATION**

(43) Date of publication:
**02.07.2025 Bulletin 2025/27**

(21) Application number: **23307436.8**

(22) Date of filing: **29.12.2023**

(51) International Patent Classification (IPC):
*A61B 5/374* (2021.01)   *A61B 5/00* (2006.01)
*A61M 16/01* (2006.01)

(52) Cooperative Patent Classification (CPC):
(C-Sets available)
**A61B 5/374; A61B 5/4821; A61B 5/7257;**
**A61M 16/024; A61M 16/104;** A61B 5/4839;
A61B 5/7267; A61M 16/0051; A61M 16/01;
A61M 2230/10; A61M 2230/30       (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Assistance Publique - Hôpitaux de**
**Paris**
**75012 Paris (FR)**

(72) Inventor: **The designation of the inventor has not**
**yet been filed**

(74) Representative: **Flesselles, Bruno F.G.**
**BF IP**
**36 rue Jean de la Fontaine**
**75016 Paris (FR)**

(54) **MONITORING ANESTHESIA**

(57)   The invention relates to methods for monitoring anesthesia of a patient and determining the proper dose of the anesthetic drug to administer to maintain a proper state of anesthesia.

**Figure 3**

EP 4 578 392 A1

Processed by Luminess, 75001 PARIS (FR)

**(Cont. next page)**

(52) Cooperative Patent Classification (CPC): (Cont.)

C-Sets
A61M 2230/10, A61M 2230/005

**Description**

The invention relates to methods for monitoring anesthesia of a patient and determining the proper dose of the anesthetic drug to administer to maintain a proper state of anesthesia.

One challenge during general anesthesia (GA) is to deliver the correct dosage of the anesthetic drug at the appropriate time.

The challenge lies in individualizing the administration of hypnotics, focusing particularly on propofol and sevoflurane. The preference for these hypnotics primarily relates to their predominantly pharmacological interaction with GABA receptors, which activate the thalamo-cortical loop.

The alpha band, an oscillation ranging from 8 to 13Hz, is one of the most notable features of the EEG (electroencephalogram) under anesthesia (notably propofol or sevoflurane anesthesia). It is also sensitive to slight changes in brain function in response to physiological modulations, which may or may not be pharmaco-induced. It was previously observed that this alpha band doesn't maintain a constant amplitude. Indeed, it can wax and wane, or simply experience transient decreases. These transient alpha decreases (TAD) have been positively linked to pre-existing cognitive fragility, as well as increased sensitivity to general anesthesia (with patients being more prone to dive too deeply during GA). No literature exists connecting this pattern to depth of anesthesia or utilizing it to adjust the quantity of drug administered.

The invention uses markers which assess brain activity captured by EEG from anesthesia, and known demographic information (age, sex, weight, etc.), to individualize the administration of hypnotics. The marker makes it possible to capture the decrease of the alpha wave amplitude, a proxy of brain function, so that adjustment of the provision of the anesthetic drug can be perform to avoid suppression.

The invention is based on the generation of new biomarker obtained from the information of the EEG that would be indicative of presence or absence of alpha wave suppression or decrease (the amplitude of the alpha wave decreasing or even disappearing). This made it possible to define a structural model that links the quantity of administered hypnotic to the brain response measured through the EEG

In a first embodiment, the invention relates to a computer-implemented method for monitoring anesthesia depth during anesthesia of a subject, where information is extracted from the alpha wave of the EEG to detect alpha suppression or alpha decrease.

The method is to be performed on a patient that is under general anesthesia. Preferably, such anesthesia has been induced and is maintained by using propofol. However, another non-opioid anesthetic drug that is administered intravenously (such as a barbiturate, a benzodiazepines, etomidate or ketamine) can have been used to induce anesthesia. The method could also be used with a patient under anesthesia induced by an opioid analgesic agent, such as alfentanil, fentanyl, remifentanil or sufentanil.

The method comprises

  a. providing data from an electroencephalogram (EEG) of the subject, wherein this data comprises the cerebral activity recorded during a time window preceding a given time point
  b. extracting alpha wave frequency information during the time window
  c. transforming the alpha wave information for obtaining amplitude values that are only positive values (absolute value)
  d. recovering the values for the maximal amplitudes for consecutive 0.05 second epochs, to obtain a list of maximal amplitude values $A_i$
  e. optionally performing a log-transform of the values of the local maxima obtained in f to obtain transformed $T_i$ values
  f. thereby obtaining a distribution of the amplitude values,
  g. optionally plotting the $A_i$ and/or the $T_i$ values with [value of $A_i$] on the x-axis and [number of $A_i$ with this value] on the y-axis
  h. modelizing the distribution of amplitude values as a mixture of two gaussian (using a gaussian mixture model with two gaussian), and obtaining the mean ($\mu$), and optionally the standard deviation ($\sigma$) and the weight (w) of each gaussian distribution.

The last step is to obtain a parameter $\Delta\mu$ associated with the distance between the two gaussian obtained after application of the gaussian mixture model. When the values $A_i$ are used, one can directly calculate the difference $\Delta\mu$ between the means (and use the absolute value to obtain a positive value). When the values $T_i$ are used (i.e. when the optional step e) has been performed), it is possible to calculate the difference between the values obtained after applying the log-inverse function to the two-gaussian means (the log-inverse being the exponential function when the log-transform uses the neperian logarithm (log or ln), or the $10^x$ function when the decimal logarithm (Log) has been used for the log-transformation).

In summary, when the $A_i$ values are used, one will obtain the value $\Delta\mu = |\mu1-\mu2|$.

When the Ti values (obtained using neperian logarithm) are used, one will obtain the value $\Delta\mu = |exp(\mu 1)-exp(\mu 2)|$ ($\mu 1$ and $\mu 2$ being the means of the first and second gaussian).

When performed continuously, the method above is repeated at consecutive time points. The method comprises

a. receiving, at consecutive time points, data from an electroencephalogram (EEG) of the subject, wherein this data comprises the cerebral activity recorded during a time window preceding a given time point

b. extracting alpha wave frequency information during the time window

c. transforming the alpha wave information for obtaining amplitude values that are only positive values (absolute value)

d. recovering the values for the maximal amplitudes for consecutive 0.05 second epochs, to obtain a list of maximal amplitude values Ai

e. optionally performing a log-transform of the values of the local maxima obtained in f

f. thereby obtaining a distribution of the amplitude values

g. optionally plotting the Ai values with [value of Ai] on the x-axis and [number of Ai with this value] on the y-axis

h. modelizing the distribution of amplitude values as a mixture of two gaussian [using a gaussian mixture model with two gaussian], and obtaining the mean, standard deviation and the weight of each gaussian distribution.

It is then possible to calculate the parameter $\Delta\mu$ as described above. Such $\Delta\mu$ parameter can be used to determine whether alpha wave suppression or alpha wave decrease has occurred and whether the patient is in a steady and proper state of anesthesia. However, if only the evolution of anesthesia is desired, it is possible also possible to calculate the difference between the means of the gaussian obtained from the Ti values (after log-transform) and to look at the variation of this value (increase of the value indicating a risk of alpha wave suppression or alpha decrease).

In the methods above, step d. indicates the recovery of the values for the maximal amplitudes for consecutive 0.05 second time windows, to obtain a list of maximal amplitude values Ai. This is because the period of the alpha waves is generally of about 0.10 second. Since the absolute value of the values has been obtained, the period of a new curve using the absolute value data would be half the initial period of the alpha wave, i.e. 0.05 second (there are generally about 20 Ai values per second, so that there are 200 Ai values for a time window of 10 seconds). This is why it is assumed that only one local minimum will be present for each 0.05 second epoch. The duration of the epoch in d. is thus half the period of the alpha wave data. As seen below, it can be calculated more precisely using the Power Spectral Density. But, as an approximation, using 0.05 second epochs is acceptable.

The methods are computer-implemented.

In a., the method comprises receiving an electroencephalogram (EEG) signal at different time points. In particular, the method shall use data from the EEG of the subject, and in particular the cerebral activity recorded during a time window preceding a given time point. In a preferred embodiment, the duration between the different time points remains identical (i.e. the EEG is received from regular time points). The method will thus evaluate the state of anesthesia for each time point. It is to be noted that, although the method uses data obtained from the human body, it does include the step of gathering the data. The method thus pertains to analysis of the data, out of the human body, and does not comprise the step of acquiring the data from the human body. The data obtained in a. is thus similar to a sample that would have been obtained from the patient, and from which information is obtained, according to the methods herein disclosed.

The EEG data that has been previously obtained from the patient is to be processed to extract information pertaining to the alpha brain waves. There is thus a need to acquire data on a time window sufficiently long to allow processing of such data and extraction of the needed information. It is thus advisable to use EEG data that has been acquired during a duration comprised between 5 and 30 seconds, preferably between 5 and 20 seconds, preferably between 5 and 15 seconds. A duration of between 10 and 15 seconds, in particular of 10 seconds is adequate.

It is preferred when consecutive time points on which the method is performed are spaced apart by a duration shorter to the duration of the time window. Although they can be spaced apart by a longer duration (for instance one, two or three times the time window), it is preferred to have such overlap to lower the inherent noise of the signal, and to obtain a continuous monitoring of the state of anesthesia (this is continuous in the sense that there is no interval during which no calculation was performed). In particular, in one embodiment, the duration between two consecutive time points on which the method is performed, is comprised between 2 and 5 seconds. In one embodiment it is 2 seconds. In another embodiment, it is 3 seconds. In another embodiment, it is 5 seconds.

After receiving the EEG data, alpha wave frequency information is extracted therefrom in step b.

The raw EEG signal (or multiple EEG signals when multiple electrodes have been positioned on the subject) can be filtered to isolate the alpha frequency band, in particular using a bandpass filter, which allows signals within the requested frequency range (the range of alpha frequency analyzed in the methods herein disclosed being between 7 and 16 Hz or or 8-16 Hz or between 7 and 14 Hz, although authors generally indicate that the alpha wave frequencies are between 7.5-13 Hz, 8-13 Hz, or 8-12 Hz, depending on the authors) to pass through while attenuating signals outside of that range. One can use any available type of filters, such as Butterworth or Chebyshev filters. One of skill in the art can select the appropriate filter design, such as the desired cutoff frequency and steepness of the filter. The cutoff frequencies and filter order are then designed, in order to encompass the alpha frequency band as defined above. The bandpass filter is then applied to the raw EEG signal. It is also possible to evaluate the quality of the filtered signal to ensure that it allows further analysis, and adjustments to the filter parameters or use of different filter designs can be envisaged if needed.

Once information pertaining to the alpha wave is extracted, the function "absolute value" is applied to the amplitudes thus obtained in c. Thus, the alpha wave information is transformed so that the amplitude values are all made positive values (absolute value). The frequency of the new data obtained is thus half the frequency of the alpha wave. Since the alphas wave frequency is about 0.10 second, the frequency of the new curve is about 0.05 second.

The local maxima (maximal amplitudes Ai) of the obtained curve are then identified. Local maximal can be obtained by determining the maxima over a half-period (since the alpha wave has been transformed by the absolute value function, the period of the resulting curve is half the period of the starting curve). Local maxima can be determined, for instance by calculating (or estimating) the derivative of the curve, wherein the maxima is obtained for a zero value of the derivative (when the derivative passes from positive to negative values). In one embodiment, the local maxima are determined for each consecutive 0.05 second epoch of the time window.

In some embodiments, the specific dominant frequency of the alpha wave can be determined, so that the epoch duration for which the local maxima are looked at can be adapted to the subject. This specific epoch duration would thus be half the period of the specific dominant frequency. In this embodiment, it is possible to calculate the Power Spectral Density (PSD) for the alpha wave spectrum. In this embodiment, the positive values obtained for the alpha wave information the time window is processed to obtain the PSD.

Thus, in this embodiment, the alpha wave frequency information is extracted by

- Filtering EEG data
- Computing EEG power for each electrode using (for instance) the fast Fourier Transform (FFT) to obtain EEG power spectral density (PSD)
- Optionally averaging the PSD for the multiple electrodes is multiple electrodes are used, to obtain a final PSD

It is reminded that the power spectral density is a measure of the power distribution across different frequencies in an EEG signal. Although one of skill in the art is aware of several methods to calculate PSD from EEG data, a widely used method is the Welch's method.

The EEG signal would be processed, in particular by filtering the data to remove frequencies outside the range of interest (here the alpha frequencies), removing or interpolating bad channels, and detrending or normalizing the data. As the studied signal is obtained from a limited time window (an epoch, as indicated above), a window function, such as the Hamming, Hanning, or Blackman window, is applied to reduce spectral leakage and improve the spectral resolution of the PSD estimate.

Other methods could be envisaged for analyzing EEG data besides FFT, such as wavelet analysis, Hilbert-Huang transform, or event-related spectral perturbation (ERSP) analysis, which involves calculating a baseline spectral power for a given frequency band (for instance, the baseline could be the values just before induction of anesthesia), and comparing this baseline to the spectral power during anesthesia.

When multiple electrodes are used to acquire the EEG data, the PSD calculated for each electrode can then be averaged to obtain the final PSD.

Using an argmax function, it is possible to identify the frequency for which the power is the highest within the frequencies that were used for analysis (preferably 7-16 Hz), and thus obtain the dominant period. Half this period can be used for identification of the local maxima, as described above.

Such isolation of the local maxima thus leads to a distribution of the maximal amplitudes Ai. Such maximal amplitudes are all positive (they are on $\mathbb{R}^{+*}$), by construction (they have been obtained after application of the absolute value function).

It is assumed that the distribution of the maximal amplitude values Ai follows a lognormal distribution. In order to obtain a distribution spanning over $\mathbb{R}$, it is advisable and preferred to perform a log-transform (using the neperian (or natural) logarithm (log or ln), or the base 10 logarithm (Log), or another logarithm function) of the values of the local maxima Ai (except the ones equal to zero, if any), to obtain transformed values Ti. Applying the logarithm function (log-transform) to the obtained distribution makes it possible to obtain a normal-like distribution.

The resulting values Ti (as well as the values Ai) can be stored in a table, together with the time on which the EEG data has been acquired.

Optionally, the values Ti or Ai can be plotted on a x-y graph, with the Ti (or Ai) values on the x-axis and the number of times such Ti (or Ai) value has been observed (number of Ti (or Ai) with this value) on the y-axis. The graph thus consists of the Ti (or Ai) values spread across the x-axis with the weight (number of each values) on the y-axis.

The next step uses the assumption that such distributions (preferably the Ti distribution obtained after Log-transform of the Ai values) are a mixture of two gaussian distributions. Using a gaussian mixture model with two gaussian distributions, it is therefore possible to modelize the distribution of amplitude values as a mixture of two gaussian.

It is thus possible, from the two obtained gaussian distribution (which together form the distribution of amplitude values), to obtain the mean ($\mu$1 and $\mu$2), standard deviation (o1 and $\sigma$2) and the weight (w1 and w2) of each gaussian distribution.

In order to determine the two gaussian from the normal distribution of the log-transformed Ti values (or from the Ai values), it is possible to use any method known ion the art. In one embodiment, such two gaussian are obtained using the Expectation-Maximization algorithm, assuming that the Ti distribution (or the Ai distribution) is a mixture of two Gaussian distributions. It is reminded that expectation-maximization (EM) algorithm is an iterative method to find (local) maximum likelihood of parameters in statistical models.

It is reminded that a Gaussian mixture model is a probabilistic model that assumes all the data points are generated from a mixture of a finite number of Gaussian distributions with unknown parameters. The Gaussian mixture model thus involves the mixture (i.e. superposition) of multiple Gaussian distributions. Rather than identifying clusters by "nearest" centroids, one can fit a set of k gaussians to the data. And it is possible estimate gaussian distribution parameters such as mean and Variance for each cluster and weight of a cluster. After learning the parameters for each data point, one can calculate the probabilities of it belonging to each of the clusters. Every distribution is multiplied by a weight to account for the fact that there is not an equal number of samples from each category.

Expectation Maximization uses first an expectation step or *E* step, which consists of calculating the expectation of the component assignments *Ck* for each data point $xi \in X$ given the model parameters $\pi k$ $\mu k$ and $\sigma k$ *mean ($\mu k$), covariance matrix ($\Sigma k$), and mixing coefficients ($\pi k$).* The maximization step or M step consists of maximizing the expectations calculated in the E step with respect to the model parameters. This step consists of updating the values $\pi k$, $\mu k$ and $\sigma k$.

The entire iterative process is repeated until the algorithm converges, giving a maximum likelihood estimate.

These EM method and algorithm are known in the art.

When performing the EM algorithm at a given time point, it is possible to use, as the parameters, the following parameters (for the Ti distribution, when the lognormal transformation has been applied)

$$\mu1 = \log(10)$$

$$\mu2 = \log(20)$$

$$w1 = w2 = 0.5$$

$$\sigma1 = \sigma2 = 5.$$

When the Ai distribution is used, the starting parameters for the EM algorithm can be

$$\mu1 = 10$$

$$\mu2 = 20$$

$$w1 = w2 = 0.5$$

$$\sigma1 = \sigma2 = 5.$$

However, one can note that, when performed continuously, the method is to be repeated, and any drift of $\Delta\mu$ (thus of the two gaussian distributions) is supposed to occur gradually, so that it is possible to use the above parameters for the first calculation, and, for the starting parameters of the EM algorithm of a later given time point, the parameters ($\mu$1, $\mu$2, w1, w2, o1, $\sigma$2) obtained for the preceding time point.

Thus, the parameters of the model parameters [mean ($\mu k$), covariance matrix ($\Sigma k$), and mixing coefficients ($\pi k$)] used at the beginning in the Expectation step of the Expectation-Maximization algorithm at a given time point are the parameters calculated for the preceding time point.

The last step consists indeed in calculating the parameter $\Delta\mu$ associated with the distance between the means ($\mu$1 and $\mu$2) of the two gaussian distributions, as indicated above. When the Ai values are used, $\Delta\mu = |\mu1-\mu2|$; when the Ti values (obtained with the neperian logarithm) are used, $\Delta\mu = |\exp(\mu1)-\exp(\mu2)|$.

It is then possible to determine whether alpha suppression is not too high (when $\Delta\mu$ is below a first threshold), but whether there is a little bit of alpha suppression (when $\Delta\mu$ is above a second threshold). This represents steady-state or stable anesthesia.

Consequently, in some embodiments, the method also comprises determining whether $\Delta\mu$ is below a first threshold and optionally above a second threshold, such difference being compared to these thresholds. As indicated, the depth of anesthesia is considered as adequate, good, proper (i.e. the subject is considered as being in a steady-state anesthesia, with minimal risks) if $\Delta\mu$ is between these thresholds.

It is preferred when the first threshold is 5 microvolts ($\mu Y$).

It is preferred when the second threshold is 0.5 microvolts.

Such method can be performed during anesthesia of a subject, therefore providing information as to the state of anesthesia of the subject in real-time. It can also be performed retrospectively (after the end of anesthesia) to determine whether the anesthesia was proper. Such information ($\mu 1$, $\mu 2$, $\sigma 1$, $\sigma 2$, w1, w2, $\Delta\mu$) can be stored in a database, together with the time on which it has been calculated, for future consideration if needed.

The method disclosed above thus makes it possible to determine whether the patient is under steady, stable and appropriate anesthesia (the patient is well asleep (will not wake up upon a surgical act) but is not in a too deep anesthesia, that may indicate a drug overdose, which could impact brain function).

It is however of interest to be able to determine the amount of anesthetic drug to administer to maintain this steady state of anesthesia.

In order to determine the dose to administer, the disclosed methods propose to take into account both the effect of the drug on the brain (as determined above) and on the MAP (as the anesthetic drug (notably propofol) has also an effect on the Mean Arterial Pressure (MAP)), using a system of differential equations based on a pharmacokinetic (PK) and pharmacodynamic (PD) models. As herein understood, the effect of the drug is the effect on the alpha wave, and it is sought to obtain anesthesia without alpha wave suppression. Such effect of the drug is thus evaluated by the $\Delta\mu$ parameter herein disclosed.

Generally, the physician will maintain an adequate level of MAP during anesthesia, and will adjust the amount of vasopressor to maintain a constant MAP (equal or above 65 mm Hg).

It is reminded that mean arterial pressure (MAP) is measured with a sphygmomanometer and is indicative of the perfusion pressure of the organs. The sphygmomanometer (or electronic electrocardioscope tensiometer) uses an oscillometric measurement. When the sleeve placed on the patient's forearm deflates automatically, oscillations are recorded by the device. These oscillations start before the actual systolic value and end after the actual diastolic value. The maximum value of the oscillation represents the mean arterial pressure. US11638529 discloses a method for estimating MAP during anesthesia, using the height of the dicrotic wave.

However, once the MAP level is achieved and maintain constant, the goal of the anesthetist is to maintain a proper anesthesia level, which corresponds to a proper sleepiness of the brain. The goal is thus to have an <u>effect of the anesthetic drug</u> (notably propofol) <u>in the brain</u>, so that the patient remains asleep (providing enough anesthetic drug), but without endangering the patient (not providing too much of the drug).

As indicated above, the $\Delta\mu$ parameter is a good indicator of the proper level of anesthesia of the brain. It is of interest to be able to determine the amount of anesthetic drug to administer to maintain the adequate level of anesthesia of the brain.

Such effect, $\Delta\mu$, which is also designed as "E" below, is dependent of a pharmacological component (amount of anesthetic drug administered, distributed in the brain, according to pharmacokinetic models), and a hemodynamic component (perfusion of the brain). With regards to the hemodynamic part, it can be noted that, when the MAP varies, there is a delay before the effect is seen in the brain (i.e. if the MAP decreases, the brain perfusion decreases after a certain delay, thereby decreasing the amount of propofol in the brain).

The present invention proposes to modelize the effect according to the Hill model. It is reminded that the Hill model refers to equations that reflect the binding of ligands to macromolecules, as a function of the ligand concentration. It can also be used in the present case for modelizing the presence of the drug in the brain (corresponding to the "binding"), as a function of the drug concentration. It assumes that the effect (binding of the ligand to the receptor) follows a sigmoid curve: there is no or little effect with no or little ligand, a saturation upon reaching a certain amount of ligand and a linear (or approximately linear) curve between two ligand concentrations.

In the present case, the effect of propofol (or of another anesthetic drug) in the brain (which is detected by the changes in the EEG) is null or low when no propofol or a small amount of such is used, reaches a saturation after a certain amount, and is somewhat linear in between.

With regards to the effect if mAP on anesthesia, this parameter represents the brain perfusion. One can easily understand that decrease in mAP leads to a lower perfusion, thus altering brain function, which is seen in the alpha wave profile. It is also assumed that the effect according to the mAP follows a sigmoid function.

The invention thus also relates to a computer-implemented method for determining the amount of anesthetic drug to administer to a subject during anesthesia to maintain proper anesthesia, comprising

a. Receiving, at a given time point, the $\Delta\mu$ information (value) calculated by a method as disclosed above,
b. Receiving at the given time point the Mean Arterial Pressure (mAP) information [value] at a time t- $\delta$ before the given time point
c. Posing the equation system

$$(1)\ \frac{dE}{dt} = -\frac{1}{\tau_P}\left(E - E_{max}(Ce)\right)$$

$$(2)\ \frac{dE}{dt} = -\frac{1}{\tau'_P}\left(E - E_{Mapmax}(mAP(t-\delta))\right)$$

wherein E corresponds to the $\Delta\mu$ information

wherein $E_{max}(Ce) = E_0 + [E_{max} - E_0] \cdot \dfrac{Ce^{\gamma}}{Ce^{\gamma} + EC_{50}^{\gamma}}$,

wherein $E_{Mapmax}(mAP) = E_{mAP0} - [E_{mAPmax} - E_{mAP0}] \cdot \dfrac{mAp^{\gamma\prime}}{mAp^{\gamma\prime} + ECmAP_{50}^{\gamma\prime}}$,

wherein Ce is the concentration of the anesthetic drug at the effect site [brain] [that is to say the concentration of propofol in the brain based on a pharmacokinetic model [in particular the Schnider model]],
$\tau p$ is a parameter corresponding to the equilibration time, $E_0$, $E_{max}$ are the minimal and maximal effects, $EC_{50}$ is the anesthetic drug concentration at which the effect is half the maximal amplitude effect ($E_{max}$-$E_0$), and $\gamma$ is the Hill coefficient (which relates to the how stiffly the effect ($\Delta\mu$) changes in response to change in Ce) $\tau'\rho$ is a parameter corresponding to the equilibration time, $E_{mAP0}$, $E_{mAPmax}$ are the minimal and maximal effects observed according when mAP varies, $EC_{mAP50}$ is the concentration at which the effect is half the maximal amplitude effect ($E_{mAPmax}$-$E_{mAP0}$), and $\gamma'$ is the Hill coefficient (which reflects how stiffly the effect ($\Delta\mu$) changes in response to change in mAP).

d. Simulating multiple E values during a given period of time using the equations and an equation solver, to simulate the value of E ($\Delta\mu$), using various Ce, and using a fixed mAP
e. Comparing the values of simulated E to a reference value of E or to a range of reference valuesof E
f. Choosing a desired brain concentration Ce of the anesthetic drug to have the E at the steady state within the desired range
g. Using a pharmacokinetic model linking the desired brain concentration to the dose to administer for a patient to determine the amount of drug to administer to obtain the calculated desired brain concentration of the anesthetic drug.

In step a., the $\Delta\mu$ value at a given time point (t) is received. This represents the state of the brain at time (t), i.e. the level of anesthesia of the brain (the effect that is of interest).

At step b., the Mean Arterial Pressure as measured or calculated at a time point t- $\delta$ (before the given time point t) is also used. It is reminded that Mean arterial pressure (MAP) is generally measured with a sphygmomanometer and is indicative of the perfusion pressure of the organs. It is also possible to measure it directly, using an intraarterial catheter, but this method is invasive and is not preferred. In some embodiments, MAP is calculated using a pulse oximeter (plethysmograph), according to the methods disclosed in US20200390347. In short, this application shows that MAP can be continuously calculated or estimated, using the height of the dicrotic wave for each heart pulse. This application also shows that any drift in the estimated MAP can be corrected, using the sphygmomanometer at regular time points, according to the current guidelines for anesthesia.

The duration $\delta$ is chosen to take into account the delay observed between the administration of the anesthetic drug and its effect on the MAP. For propofol, it is generally about 6-7 minutes, thus one can choose $\delta$ comprised between 3 and 10 minutes, preferably between 5 and 8 minutes.

As indicated above, it is postulated that the effect of the drug in the brain (E, which is actually represented by the parameter $\Delta\mu$ calculated above which represents the state of brain anesthesia) varies, depending on the pharmacologic model and of the perfusion, and also depends on the effect at time t.

It is thus possible to pose the following equation.

$$(1)\ \frac{dE}{dt} = -\frac{1}{\tau_P}\left(E - E_{max}(Ce)\right)$$

$$(2)\ \frac{dE}{dt} = -\frac{1}{\tau'_P}\left(E - E_{Mapmax}(mAP(t-\delta))\right)$$

wherein E corresponds to the effect that one wants to observe (the $\Delta\mu$ information).

Solving this equation system (see below) makes it possible to determine how the $\Delta\mu$ will vary, depending on the concentration of the anesthetic drug at the effect site (the brain).

$$E_{max}(Ce) = E_0 + [E_{max} - E_0] \cdot \frac{Ce^\gamma}{Ce^\gamma + EC_{50}^\gamma}$$

In equation (1) of this system, . This equation is the Hill equation, and corresponds to the dose-effect equation of a drug (Hill's equation).

Equation (1) thus reflects that the effect E varies depending on the concentration of the drug, and of the effect at time t. Thus, Ce is the concentration of the anesthetic drug at the effect site (in the brain), $E_0$, $E_{max}$ are the minimal and maximal effects, ECso is the concentration at which the effect is half the maximal amplitude effect ($E_{max}$-$E_0$), and $\gamma$ is the Hill coefficient [which relates to the how stiffly the effect ($\Delta\mu$) changes in response to change in Ce]. $EC_{50}$ is herein written using the formalism of the Hill equation and actually corresponding to Ceso.

_Ce_ thus corresponds to the concentration of the anesthetic drug in the brain, that is to say the concentration of the drug (usually propofol) in the brain based on a pharmacokinetic model. One can use the Schnider or the Marsh model. The Marsh and Schnider models are pharmacokinetic models for determining propofol concentration in the various compartments. The Marsh model has been adapted from the Gepts model, in which the rate constants are fixed, whereas compartment volumes and clearances are weight proportional.

The Schnider model was developed during combined pharmacokinetic-pharmacodynamic modelling studies. It has fixed values for V1, V3, k13, and k31, adjusts V2, k12, and k21 for age, and adjusts k10 (elimination rate constant) according to total weight, lean body mass (LBM), and height. It was derived during a combined pharmacokinetic and pharmacodynamic study in a single set of 24 volunteers (Schnider et al, Anesthesiology, 1998, 88, 1170-82; Schnider et al, Anesthesiology, 1999, 90, 1502-16), using total body weight, age, height, and lean body mass (LBM) (calculated from total weight, gender, and height) as co-variates.

These models are known in the art, and allow to determine the dose of propofol to administer to obtain a desired effect in the brain.

They are used by physicians to determine the initial dose of propofol to administer to a patient to induce anesthesia, by defining a target concentration to reach in the brain of the patient at the onset of anesthesia, the dosage of propofol being determined by the model.

$$E_{Mapmax}(mAP) = E_{mAP0} - [E_{mAPmax} - E_{mAP0}] \cdot \frac{mAp^{\gamma'}}{mAp^{\gamma'} + ECmAP_{50}^{\gamma'}}$$

In equation (2), . This equation (2) reflects the variation of the effect of the drug in the brain, depending on the MAP. However, as indicated, in general, the MAP will not vary much overtime.

The various coefficients, $E_0$, $E_{max}$, $EC_{50}$, $\gamma$, $\tau_P$, $E_{mAP0}$, $E_{mAPmax}$, $EC_{mAP50}$, $\gamma'$, $\tau_P'$ can be determined as follows.

One uses a population of patients for whom the values Ce, MAP and E (delta mu) are available. Consequently, for this population of patients, the information pertaining both to the concentration of the anesthetic drug and the anesthesia state of the brain is available, as well as the MAP. It is to be noted that, for a given patient, it is possible to have multiple data (Ce, E, MAP), measured over time. It is also noted that Ce is obtained for the Schnider equation, as indicated above, having knowledge of the amount of drug provided (concentration of the vial and administration rate).

It is preferred to have at least 250 patients in the population, with a good distribution of age, weight, height, sex and comorbidity (diabetes, hypertension, history of heart condition, neurological or cardiac treatment).

A Hamiltonian Monte Carlo (HMC) algorithm or variational inference to calculate the various coefficients.

The above coefficients (Eo, $E_{max}$, ECso, $\gamma$, $\tau_P$, $E_{mAP0}$, $E_{mAPmax}$, $EC_{mAP50}$, $\gamma'$, $\tau_P'$) could be calculated patient by patient (unpooled). But the values may be very different from one another, as they may depend on the characteristics of the patient (age, sex, weight, BMI, comorbidity). Alternatively, the coefficients could be calculated for all available data (pooled calculation). But this method may lead to a loss of the patients' characteristics.

It is thus preferable to assume that the coefficients are patient-specific, but that each corresponds to a particular distribution (a Gaussian distribution) except for $\tau_P$, which is assumed to have a lognormal distribution). The HMC is thus configured to find and converge to the coefficients in this kind of distribution. Thus, the HMC (or the variational inference algorithms are constrained, so that the values that are too far from the center of the distribution are considered to have a low probability of fitting the model. This kind of search is called perform a partially pooled (hierarchical) search and eventually provides

- values of $E_0$, $E_{max}$, $EC_{50}$, $\gamma$, $\tau_P$, $E_{mAP0}$, $E_{mAPmax}$, $EC_{mAP50}$, $\gamma'$, $\tau_P'$ for each patient;
- the general distribution of $E_0$, Emax, $EC_{50}$, $\gamma$, $\tau_P$, $E_{mAP0}$, $E_{mAPmax}$, $EC_{mAP50}$, $\gamma'$, $\tau_P'$ among the patients' population.

It is reminded that the methods for obtaining such information from the data of the population of patients (HMC, variational inference) are known in the art, to build models from existing data.

The following step would be to build a model (notably using linear regression, or a learning machine classifier) to be able to obtain the various parameters, for a given patient, from the patient's physiological values.

The model may be obtained using linear discriminant analysis, a neural network, clustering techniques, support vector machine, logistic regression, naive Bayes, random forest, decision tree, gradient boosting or the like. One can use a machine learning classifier which is an artificial neural network, such as a feedforward neural network, a convolutional neural network, or a recurrent neural network. Using a convolutional neural network is of particular interest.

In some embodiments, one can use $E_o = 0$ and $E_{max} = 0.5$. One can also use 3.35ug/ml as a starting concentration for $EC_{so}$, and 1.24 for the Hill coefficient ($\gamma$).

For the initial conditions, one can use $E_{mAP0} = 0$, $E_{mAPmax} = 1$, $EC_{mAP50}$ 75 and $\gamma' = 5$.

When performing the method for identifying the adequate anesthetic drug dosage described above, it is thus understood that the parameters $E_o$, $E_{max}$, $EC_{50}$, $\gamma$, $\tau_P$, $E_{mAP0}$, $E_{mAPmax}$, $EC_{mAP50}$, $\gamma'$, $\tau_P'$ are available for equations (1) and (2), calculated from the physiological characteristics of the patient (notably age, sex, BMI, weight, and/or comorbidities) according to the methods described above.

In summary, $E_0$, $E_{max}$, $EC_{so}$, $\gamma$, $\tau_P$, $E_{mAP0}$, $E_{mAPmax}$, $EC_{mAP50}$, $\gamma'$, $\tau_P$ have been calculated from a model (the regression model or the machine learning classifier model) obtained from a population of patients, wherein the $E_0$, $E_{max}$, $EC_{50}$, $\gamma$, $\tau_P$, $E_{mAP0}$, $E_{mAPmax}$, $EC_{mAP50}$, $\gamma'$, $\tau_P'$ for the patients were determined by a Hamilton Monte Carlo method.

Equation (1) and (2) can thus be "solved", meaning that it is possible to simulate multiple E values during a given period of time (the following 20-30 minutes: it is also possible to use a duration of at least five times the highest between $\tau$ and $\delta$) using the equations (1) and (2) and an equation solver for solving the equation system. One can use any available method for solving the equation system (1) and (2) disclosed above. In particular, one can use a Runge-Kutta 4 solver, the Euler method (forward Euler method) or the Heun's method.

This makes it possible to simulate the steady state of E ($\Delta\mu$), by using various different Ce (it is understood that mAP is considered to be invariant, as the anesthetist ensures that such value remains constant during anesthesia, or that the variation of mAP observed at the block doesn't impact brain perfusion).

Thus, a series of values (simulated $\Delta\mu$ at steady state depending on Ce) are obtained. The simulated E (Effect value, $\Delta\mu$) can be compared to a reference (target value or desired value) or to a range of reference values. It is thus possible to see whether the effect remains within the desired values (0.5 $\mu$V and 5 $\mu$V) depending on the anesthetic drug concentration.

It is thus possible to identify adequate and desired brain concentration Ce of the anesthetic drug to obtain a value E ($\Delta\mu$) at the steady state within the desired range. Using the pharmacokinetic model (Schnider or Marsh model), it is possible to determine the amount of drug to administer to obtain the calculated desired brain concentration of the anesthetic drug.

The above methods are of particular interest when anesthesia is induced by propofol as the anesthetic drug.

The invention also relates to a method for administering an anesthetic drug to a subject, comprising

a) Performing a method as disclosed above, during anesthesia of the subject
b) Adjusting the rate of administration of the anesthetic drug to the subject to obtain the calculated desired brain concentration of the anesthetic drug.

The invention also relates to a device for performing the method for monitoring anesthesia depth during anesthesia of a subject, comprising a processing unit comprising at least one processor operatively coupled to the at least one electrode

a) a processing unit comprising at least one processor operatively coupled to at least one electrode, in order to receive the EEG signal of the subject captured by the electrode
b) at least one non-transitory computer-readable medium comprising program instructions that, when executed by the at least one processor, causes the device to:

i. obtain the EEG signal of the subject captured by the electrode
ii. at (regular) consecutive time points, isolate the EEG signal obtained during a time window preceding the given time point
iii. Extract alpha wave frequency information within the time window
iv. Transform the alpha wave information by applying the absolute value function to obtain a curve of amplitudes containing only positive values
v. Identifying the local maxima Ai of the obtained positive values
vi. Optionally perform a log-transform of the Ai values to obtain Ti values

vii. Modelize the distribution of the Ti values values as a mixture of two gaussian, and obtaining the means ($\mu$1 and $\mu$2), standard deviations and the weight of each gaussian distribution
viii. Calculate the parameter $\Delta\mu$

c) Optionally a monitor/screen to display the calculated $\Delta\mu$ and optionally a warning [a visual and/or sound signal] if such $\Delta\mu$ is above a first threshold or below a second threshold.

The device may also send EEG signal data, Ai, Ti, means ($\mu$1 and $\mu$2), standard deviations, weight, $\Delta\mu$ information to a storage database, so that they are time-stamped for recording the information for potential future analysis.

The invention also relates to a device for calculating for determining the amount of anesthetic drug to administer to a subject during anesthesia to maintain proper anesthesia, comprising

a. a processing unit comprising at least one processor
b. at least one non-transitory computer-readable medium comprising program instructions that, when executed by the at least one processor, causes the device to:

i. Receive, at a given time point, the $\Delta\mu$ information as herein disclosed,
ii. Receive at the given time point the Mean Arterial Pressure (mAP) information at a time t- $\delta$ before the given time point
iii. Solving the equation system

$$(1)\ \frac{dE}{dt} = -\frac{1}{\tau_P}\left(E - E_{max}(Ce)\right)$$

$$(2)\ \frac{dE}{dt} = -\frac{1}{\tau_P}\left(E - E_{max}(mAP(t - \delta))\right)$$

as disclosed above to calculate values of E ($\Delta\mu$) according to various inputted Ce, and determining the concentration of anesthetic drug to provide to obtain a target $\Delta\mu$ or $\Delta\mu$ within a given range.

As indicated above, iii may be performed by simulating multiple E values during a given period of time (15-20 minutes) using various Ce, and using a fixed mAP, comparing the values of simulated E to a reference value of E or to a range of reference values of E, choosing a desired brain concentration Ce of the anesthetic drug to have the E at the steady state within the desired range, using a pharmacokinetic model linking the desired brain concentration to the dose to administer for a patient to determine the amount of drug to administer to obtain the calculated desired brain concentration of the anesthetic drug.

Optionally, the device further comprises a syringe pump for administering the anesthetic drug to the subject according to the calculated amount.

## Figures

Figure 1: A. Representation of the alpha wave after filtering. The local extrema are indicated by arrows. B. Distribution of the local maxima after log-transform. The distribution is then considered to be a mixture of two gaussians. C. representation of various alpha wave situations. D. Two gaussian distributions, depending on the alpha wave situation of C.
Figure 2: representation of the delay in the brain effect upon drop in mAP.
Figure 3: representation of $\Delta\mu$ (gTAD) as measured and as predicted overtime.

## Examples

### Example 1. Position of the problem

For an anesthesist, it is important to both monitor the anesthesia and to delivering the correct dosage at the appropriate time.
A challenge lies in individualizing the administration of hypnotics, focusing particularly on propofol and sevoflurane. The preference for these hypnotics primarily relates to their predominantly pharmacological interaction with GABA receptors, which activate the thalamo-cortical loop.

This aspect is of interest as, during general anesthesia, the characteristics of thalamo-cortical loop activation, and to a degree, the quality of anesthesia, can be real-time monitored using the electroencephalogram (EEG) method, especially the alpha band (8-13Hz), from which biomarkers will be derived from.

A strategy is proposed that involves assessing brain activity captured by EEG in the operating room, supplemented with known demographic information (age, sex, weight, etc.), to individualize the administration of hypnotics.

The solution is founded on four key components:

a) The generation of a series of digital biomarkers calculated from the EEG, with particular emphasis on $\Delta\mu$, a novel biomarker.

b) A structural model that links the quantity of administered hypnotic to the brain response measured through the EEG.

c) A computational method that have potentiality to tailor the model for each patient in the operating room.

**The type of data used:**

The solution employs a variety of data types, including:

- Demographic and morphological data: Parameters such as age, weight, height, sex, presence of hypertension or diabetes, and long-term prescription of benzodiazepines are taken into account.
- EEG data gathered during anesthesia: This involves signals from the Fp2 electrode, measured in microvolts and collected with a minimum sampling frequency of 63Hz.
- Administered amount of propofol: the cumulative quantity injected, target controlled infusion (TCI), or minimum alveolar concentration (MAC) is tracked.
- Measurement of mean arterial blood pressure (mAP or MAP) during general anesthesia: This measurement could be invasive, reconstructed, or non-invasive.

**Example 2. Methods**

a) Biomarker computation

The model serves to associate the quantity of drug administered with its effect on the body, particularly the brain, which is the primary site targeted by hypnotics.

Currently, brain function is monitored using a Depth-of-Anesthesia monitor, which employs a simplified EEG. The simplification involves the use of only a few electrodes (1 to 4), positioned on frontal sub-hairline regions. Typically, one can utilize the electrode at the Fp2 position. The signal is recorded at a sampling frequency of at least 63Hz, a value influenced by historic results obtained with Masimo monitors and chosen to accurately represent beta and alpha frequencies.

The alpha band, an oscillation ranging from 8 to 13Hz, is one of the most notable features of the EEG under propofol or sevoflurane anesthesia. It is also sensitive to slight changes in brain function in response to physiological modulations, which may or may not be pharmaco-induced.

It was previously observed that this alpha band doesn't maintain a constant amplitude. Indeed, it can wax and wane, or simply experience transient decreases. These transient alpha decreases (TAD) have been positively linked to pre-existing cognitive fragility [1], as well as increased sensitivity to general anesthesia ([2], with patients being more prone to dive too deeply during GA).

Previous studies employed a method based on various threshold optimizations and a 'contrast function' used to identify periods of TAD. This method had several limitations.

In particular, it was not extendable to drugs like sevoflurane and was highly dependent on the choice of threshold, which also hindered its robustness against external artifacts.

Here, a marker is defined according to the patient's EEG only.

A Gaussian mixture model was used for the statistical approach. Specifically, a 2 Gaussian model was used where the Gaussian with the smaller mean characterizes the TAD, and the one with larger values signifies the alpha band with 'normal' amplitudes.

This Gaussian-based representation of TAD is denoted as gTAD in this document, standing for Gaussian TAD.

The computational process to obtain this biomarker is as follows.

Firstly, the raw (unprocessed) EEG signal is filtered to isolate the alpha band. This can be achieved either through a Daubechies wavelet decomposition or using traditional filter techniques to yield only 8-13Hz oscillations (larger range such as 7-16Hz can also be used).

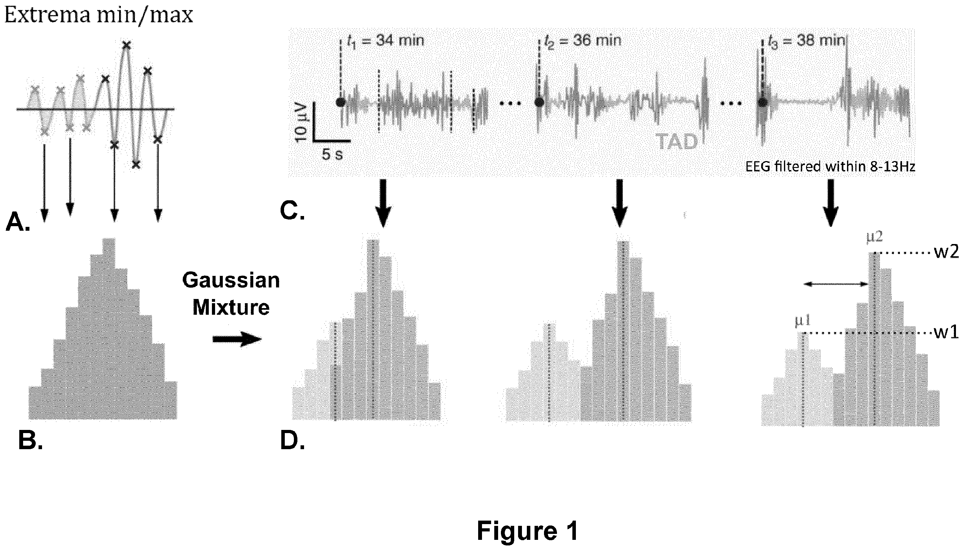

Once the signal is filtered (Figure 1.A), the goal was to obtain a measurement of the amplitudes present in the signal.

This was achieved by applying the absolute value function to the signal, thus turning minima into maxima.

Subsequently, a function was used to search for local maxima, setting the distance between two maxima to be half the period computed from the patient's dominant alpha band frequency f0. Such dominant alpha band frequency f0 and search for the local maxima can be directly computed from the patient's EEG, using an argmax function on the power spectral density (PSD) for frequencies between 8 to 13Hz.

The Welch method was used to compute the PSD.

An alternative solution to derive f0 was also used, based on a statistical model that predicts f0 based on patient characteristics (approximation).

Ultimately, the maxima on the positive representation of the alpha band as well as their associated time were obtained (Figure 1.A).

These maxima were then represented as a distribution (Figure 1.B).

Since all maxima values are by construction positive, a log-normal distribution was assumed, which prompted the use of a log-transform to achieve a normal-like distribution, or at least a distribution with values spread across the entire real axis.

This step was considered as favorable for applying the Gaussian mixture model, as the Gaussian probability density function support (permissible x values) also belongs to the real axis.

The Gaussian mixture problem on these log-transformed values was solved by determining the most probable mean and variance of two Gaussian distributions, which, when superimposed, best mimic the empirical distribution measured.

Biomarker extraction from this method considered parameters such as the mean of each Gaussian ($\mu 1$ and $\mu 2$), the associated probability for each Gaussian mode (w1, w2-the weights of the mixture), and the variance of each Gaussian ($\sigma 1$ and $\sigma 2$).

These features are illustrated in Fig. 1.D, corresponding to the EEG represented in Figure 1.C. From a physiological standpoint, $\mu 1$ represents the dominant TAD amplitude for a given patient, while $\mu 2$ signifies the normal, non-TAD amplitude of the alpha band.

However, the mere existence of TAD is conditional on w1, w2 and on the difference between $\mu 1$ and $\mu 2$.

Indeed, negligible w1 or small difference between $\mu 1$ and $\mu 2$ indicate that the Gaussian mixture is essentially a single Gaussian model, meaning no TAD.

A parameter $\Delta\mu$ was calculated as the difference between the exponential values of $\mu 1$ and $\mu 2$ (since a log-transform was performed).

The method was repeated using signal epochs (15 to 30s) that overlapped.

The Gaussian mixture was estimated over these epochs, producing a time series as a result, and drift of the difference between $\mu 1$ and $\mu 2$ or of $\Delta\mu$ could be assessed, thereby showing appearance of TAD.

b) The structural equation

The goal of the structural equations is to establish a connection between the amount of propofol administered, the intra-operative blood pressure, and the effect on the brain, as summarized in section a) above.

In the case, it was assumed that the structural equation comprises a system of differential equations based on a pharmacokinetic (PK) and pharmacodynamic (PD) models.

The data primarily focus on propofol as the main hypnotic, though these results can be extended to other hypnotics, notably sevoflurane.

The structural equation can be divided into four sub-models:

- a pharmacokinetic (PK) model for propofol
- a pharmacodynamic model for propofol's impact on brain function
- and the effect of blood pressure on brain function.

These models will be referred to as models 1, 2, and 3.

Several propofol PK models exist in the literature, including the Schnider [3], Marsh, and Eleveld [4] models.

The PK model **(model 1)** was based on the Schnider model, which is already routinely used in operating theaters for total intravenous propofol anesthesia. This system is a four-compartmental mamillary model, where the drug is injected into a central compartment and distributed into two auxiliary ones. The fourth compartment represents the brain, with propofol concentration passively equilibrating with the central compartments. In our approach, we have implemented a modification to this model by adding a dynamic element to the elimination constant. This adjustment is proposed to account for scenarios where the rate at which the body purges the drugs might deteriorate during the procedure, leading to accumulation.

The propofol pharmacodynamics **(model 2)** effect consists in the association of a first order exponential dynamic with an $E_{max}$ model:

$$(1)\ \frac{dE}{dt} = -\frac{1}{\tau_P}\left(E - E_{max}(Ce)\right),$$

With $E_{max}(Ce) = E_0 + [E_{max} - E_0] \cdot \frac{Ce^\gamma}{Ce^\gamma + EC_{50}^\gamma}.$

The parameters above are as defined in the specification. One can note that equation (1) is similar to the charge equation of a capacitor. It is indeed assumed that there is some latency in the propofol concentration in the brain, and that the variation of the effect is associated with the actual effect.

Similarly, it was proposed a similar approach to account for the effect of mean arterial pressure on the brain function **(model 3),** except in this case, a delay between the blood pressure modulation and the change in brain function was accounted for:

$$(2)\ \frac{dE}{dt} = -\frac{1}{\tau'_P}\left(E - E_{Mapmax}(mAP(t - \delta))\right)$$

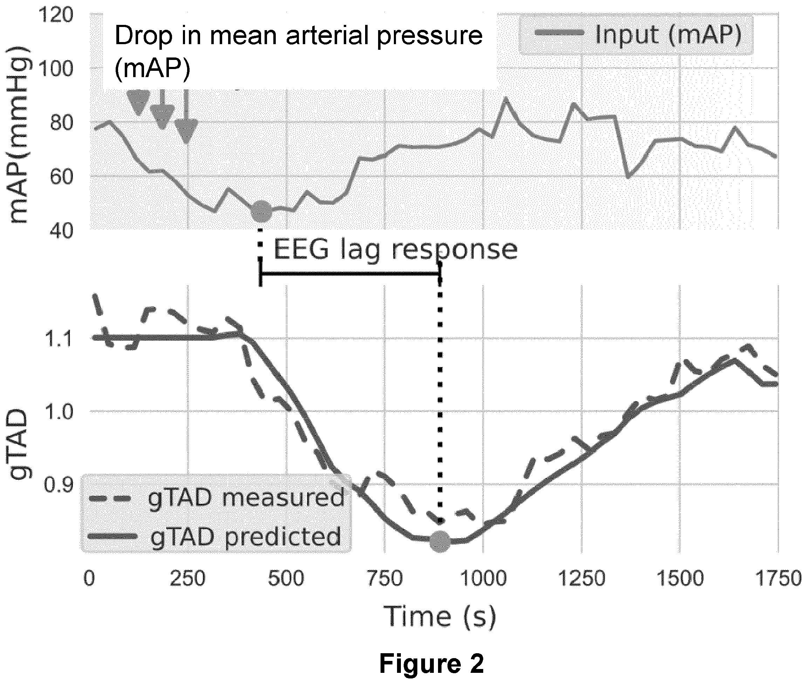

With $E_{Mapmax}(mAP) = E_{mAP0} - [E_{mAPmax} - E_{mAP0}] \cdot \frac{mAp^{\gamma'}}{mAp^{\gamma'} + ECmAP_{50}^{\gamma'}},$ where $\delta$ is a delay (as seen in Figure 2, there is a delay in the effect seen in the brain after drop of mAP). The parameters are as described in the specification. The function has the same structure as the one used for **model 2.**

The effect is ultimately regarded as a superposition of the effects computed from **models 2** and **3.**

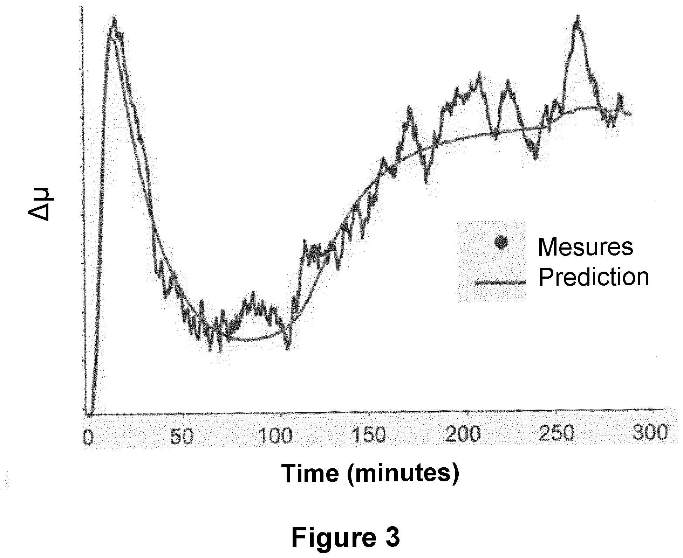

Figure 3 shows that the model makes it possible to predict the variation of the $\Delta\mu$ (light gray line) which corresponds to the actually measured $\Delta\mu$ (dark gray line) observed when propofol concentration varies.

c) Integration within a clinical protocol

The above markers and methods can provide information about the amount of propofol to administer without causing any interruptions to the procedure.

Prior to general anesthesia, patient data are used to estimate the best set of parameters. Then, during the induction period, measurements such as mean arterial pressure (mAP), the amount of drug administered, and EEG readings were fed into the model for fine-tuning.

Throughout the entire intervention, these parameters were continuously updated.

As a result, the model is statistically consistent with the patient's characteristics and continues to improve during the intervention.

The interaction between the anesthesiologist and the model primarily occurs through the proposed target-controlled infusion of propofol to achieve a specific effect on brain response. In other words, given a desired state of brain function (which can either be proposed or enforced), the model can determine how to administer the hypnotic (what amount) to reach such an effect.

Such approach is already in routine use in the OR, where syringe pumps have the Schnider model embedded in their systems. This allows the anesthesiologist to set a desired target of propofol concentration in the brain, and the syringe pump estimates how much of the product must be pushed.

The strategy here is similar except that the target is not an a *priori* concentration in the brain but the expected effect in the brain, which is an accurate estimation of the patient's depth of anesthesia, where the model transforms the expected effect in the brain to the amount of anesthetic drug to provide.

References

[1]J. Cartailler, C. Touchard, P. Parutto, E. Gayat, C. Paquet, and F. Vallée, "Brain fragility among middle-aged and elderly patients from electroencephalogram during induction of anaesthesia," Eur. J. Anaesthesiol. EJA, vol. 38, no. 12, pp. 1304-1306, Dec. 2021, doi: 10.1097/EJA.0000000000001524.
[2] J. Cartailler, P. Parutto, C. Touchard, F. Vallée, and D. Holcman, "Alpha rhythm collapse predicts iso-electric suppressions during anesthesia," Commun. Biol., vol. 2, no. 1, pp. 1-10, 2019.

[3] A. R. Absalom and K. P. Mason, Total intravenous anesthesia and target controlled infusions. Springer, 2017.

[4] D. J. Eleveld, P. Colin, A. R. Absalom, and M. M. R. F. Struys, "Pharmacokinetic-pharmacodynamic model for propofol for broad application in anaesthesia and sedation," Br. J. Anaesth., vol. 120, no. 5, pp. 942-959, May 2018, doi: 10.1016/j.bja.2018.01.018.

[5] P. L. S. Chan, P. Jacqmin, M. Lavielle, L. McFadyen, and B. Weatherley, "The use of the SAEM algorithm in MONOLIX software for estimation of population pharmacokinetic-pharmacodynamic-viral dynamics parameters of maraviroc in asymptomatic HIV subjects," J. Pharmacokinet. Pharmacodyn., vol. 38, no. 1, pp. 41-61, Feb. 2011, doi: 10.1007/s10928-010-9175-z.

[6] G. Papamakarios, E. Nalisnick, D. J. Rezende, S. Mohamed, and B. Lakshminarayanan, "Normalizing flows for probabilistic modeling and inference," J. Mach. Learn. Res., vol. 22, no. 1, pp. 2617-2680, 2021.

[7]A. Gelman, J. B. Carlin, H. S. Stern, D. B. Dunson, A. Vehtari, and D. B. Rubin, Bayesian Data Analysis, Third Edition. CRC Press, 2013.

[8] N. Kantas, A. Doucet, S. S. Singh, J. Maciejowski, and N. Chopin, "On Particle Methods for Parameter Estimation in State-Space Models," Stat. Sci., vol. 30, no. 3, Aug. 2015, doi: 10.1214/14-STS511.

**Claims**

1. A computer-implemented method for monitoring anesthesia depth of a subject at a given time point, comprising

   a. providing data from an electroencephalogram (EEG) of the subject, wherein this data comprises the cerebral activity recorded during a time window preceding the given time point
   b. extracting alpha wave frequency information during the time window
   c. transforming the alpha wave information for obtaining amplitude values that are only positive values (absolute value)
   d. recovering the values for the maximal amplitudes for consecutive epochs, wherein the epoch duration is half the period of the alpha wave signal, to obtain a list of maximal amplitude values Ai
   e. optionally performing a log-transform of the values of the local maxima obtained in f to obtain transformed Ti values
   f. thereby obtaining a distribution of the amplitude values,
   g. optionally plotting the Ai and/or the Ti values with [value of Ai] on the x-axis and [number of Ai with this value] on the y-axis
   h. modelizing the distribution of amplitude values as a mixture of two gaussian (using a gaussian mixture model with two gaussian), and obtaining the mean ($\mu$), and optionally the standard deviation ($\sigma$) and the weight (w) of each gaussian distribution.

2. The method of claim 1, further comprising calculated a parameter $\Delta\mu$ associated with the distance between the means ($\mu 1$ and $\mu 2$) of the two gaussian distributions, as indicated above, wherein $\Delta\mu = |\mu 1-\mu 2|$ when Ai values are used and wherein $\Delta\mu = |\exp(\mu 1)-\exp(\mu 2)|$ when Ti values obtained with the neperian logarithm are used.

3. The method of claim 2, wherein the anesthesia is considered as adequate if the $\Delta\mu$ is below a first threshold and above a second threshold.

4. The method of claim 3, wherein the first threshold is 5 microvolts and the second threshold is 0.5 microvolt.

5. The method of any one of claims 1 to 4, wherein the distribution of amplitude values as mixture of two gaussian is performed using the Expectation-Maximization algorithm.

6. The method of any one of claims 1 to 5, wherein the time window is between 5 and 30s seconds.

7. The method of any one of claims 5 to 6, which is repeated at consecutive time points.

8. The method of claim 7, wherein the duration between two consecutive time points is comprised between 2 and 5 seconds.

9. The method of claim 7 or 8, wherein the distribution of amplitude values as mixture of two gaussian is performed using the Expectation-Maximization algorithm and wherein the parameters of the model parameters [mean ($\mu k$), covariance matrix ($\Sigma k$), and mixing coefficients ($\pi k$)] used at a given time point are the parameters calculated for the preceding time point.

10. A computer-implemented method for determining the amount of anesthetic drug to administer to a subject during anesthesia to maintain proper anesthesia, comprising

 a. Receiving, at a given time point, the $\Delta\mu$ information calculated by a method according to any one of claims 1 to 9,
 b. Receiving at the given time point the Mean Arterial Pressure (mAP) information at a time t- $\delta$ before the given time point
 c. Posing the equation system

$$(1)\ \frac{dE}{dt} = -\frac{1}{\tau_P}\left(E - E_{max}(Ce)\right)$$

$$(2)\ \frac{dE}{dt} = -\frac{1}{\tau'_P}\left(E - E_{Mapmax}(mAP(t-\delta))\right)$$

 wherein E corresponds to the $\Delta\mu$ information

$$E_{max}(Ce) = E_0 + [E_{max} - E_0] \cdot \frac{Ce^\gamma}{Ce^\gamma + EC_{50}^\gamma},$$

 wherein
 wherein $E_{Mapmax}(mAP) = E_{mAP0} - [E_{mAPmax} - E_{mAP0}] \cdot$
 wherein Ce is the concentration of the anesthetic drug in the brain,
 $\tau p$ is a parameter corresponding to the equilibration time, $E_0$, $E_{max}$ are the minimal and maximal effects of the drug in the brain, $EC_{50}$ is the anesthetic drug concentration at which the effect is half the maximal amplitude effect ($E_{max}$-$E_0$), and $\gamma$ is the Hill coefficient associated with the change of the effect in response to change in Ce
 $\tau'p$ is a parameter corresponding to the equilibration time, $E_{mAP0}$, $E_{mAPmax}$ are the minimal and maximal effects observed according when mAP varies, $EC_{mAP50}$ is the concentration at which the effect is half the maximal amplitude effect ($E_{mAPmax}$-$E_{mAP0}$), and $\gamma'$ is the Hill coefficient associated with the change of the effect in response to variation of mAP.

 d. Using various Ce, simulating multiple E values during a given period of time using the equations and an equation solver, to simulate the value of E ($\Delta\mu$) and using a fixed mAP
 e. Comparing the values of simulated E to a reference value of E or to a range of reference values of E
 f. Choosing a desired brain concentration Ce of the anesthetic drug to have the E at the steady state within the desired range
 g. Using a pharmacokinetic model linking the desired brain concentration to the dose to administer for a patient to determine the amount of drug to administer to obtain the calculated desired brain concentration of the anesthetic drug.

11. The method of claim 10, wherein $\delta$ is comprised between 3 and 10 minutes, preferably between 5 and 8 minutes.

12. The method of claim 10 or 11, wherein the pharmacokinetic model is the Schnider model.

13. The method of any one of claims 10 to 12, wherein the equation solver is a Runge-Kutta 4 solver.

14. The method of any one of claims 10 to 13, wherein $E_0$, $E_{max}$, $EC_{50}$, $\gamma$, $\tau_P$, $E_{mAP0}$, $E_{mAPmax}$, $EC_{mAP50}$, $\gamma'$, $\tau_P'$ have been calculated from a model obtained from a population of patients, wherein the $E_0$, $E_{max}$, $EC_{50}$, $\gamma$, $\tau_P$, $E_{mAP0}$, $E_{mAPmax}$, $EC_{mAP50}$, $\gamma'$, $\tau_P'$ for the patients were determined by a Hamilton Monte Carlo method.

15. The method of any one of claims 1 to 14, wherein the drug used for anesthesia is propofol or sevoflurane.

16. A device for performing the method according to any one of claims 1 to 9, comprising a processing unit comprising at least one processor operatively coupled to the at least one electrode

 a) a processing unit comprising at least one processor operatively coupled to at least one electrode, in order to receive the EEG signal of the subject captured by the electrode

b) at least one non-transitory computer-readable medium comprising program instructions that, when executed by the at least one processor, causes the device to:

    i. obtain the EEG signal of the subject captured by the electrode

    ii. at (regular) consecutive time points, isolate the EEG signal obtained during a time window preceding the given time point

    iii. Extract alpha wave frequency information within the time window

    iv. Transform the alpha wave information by applying the absolute value function to obtain a curve of amplitudes containing only positive values

    v. Identifying the local maxima Ai of the obtained positive values

    vi. Optionally perform a log-transform of the Ai values to obtain Ti values

    vii. Modelize the distribution of the Ti values values as a mixture of two gaussian, and obtaining the means ($\mu 1$ and $\mu 2$), standard deviations and the weight of each gaussian distribution

    viii. Calculate the parameter $\Delta\mu$

c) Optionally a monitor/screen to display the calculated $\Delta\mu$ and optionally a warning signal if such $\Delta\mu$ is above a first threshold or below a second threshold.

**17.** A device for performing the method according to any one of claims 10 to 15, comprising

a. a processing unit comprising at least one processor

b. at least one non-transitory computer-readable medium comprising program instructions that, when executed by the at least one processor, causes the device to:

    i. Receive, at a given time point, the $\Delta\mu$ information as herein disclosed,

    ii. Receive at the given time point the Mean Arterial Pressure (mAP) information at a time $t-\delta$ before the given time point

    iii. Solving the equation system

$$(1) \; \frac{dE}{dt} = -\frac{1}{\tau_P}\left(E - E_{max}(Ce)\right)$$

$$(2) \; \frac{dE}{dt} = -\frac{1}{\tau_P}\left(E - E_{max}(mAP(t-\delta))\right)$$

as disclosed above to calculate values of E ($\Delta\mu$) according to various inputted Ce, and

    iv. determining the concentration of anesthetic drug to provide to obtain a target $\Delta\mu$ or a $\Delta\mu$ within a given range.

**Figure 1**

Figure 2

Figure 3

Europäisches
Patentamt

European
Patent Office

Office européen
des brevets

**EUROPEAN SEARCH REPORT**

Application Number

EP 23 30 7436

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Toker Daniel ET AL: "Spectral EEG correlations from the dfferent phases of general anesthesia", Frontiers in Medicine. 2023 Mar 6;10:1009434., 6 March 2023 (2023-03-06), pages 1-15, XP093174299, DOI: 10.3389/fmed.2023.1009434 Retrieved from the Internet: URL:https://www.ncbi.nlm.nih.gov/pmc/articles/PMC10025404/pdf/fmed-10-1009434.pdf [retrieved on 2024-06-13] | 1-9,16 | INV. A61B5/374 A61B5/00 A61M16/01 |
| A | * abstract * <br> * sect.2.1, 2.2, 2.4, 3.2 * <br> * sect. "statistical analysis" * <br> * eq.3 * <br> * figures 1,2 * <br> * the whole document * | 10-15,17 | |
| X,D | CARTAILLER J?R?ME ET AL: "Alpha rhythm collapse predicts iso-electric suppressions during anesthesia", COMMUNICATIONS BIOLOGY, [Online] vol. 2, no. 1, 1 December 2019 (2019-12-01), XP055873174, DOI: 10.1038/s42003-019-0575-3 Retrieved from the Internet: URL:https://www.nature.com/articles/s42003-019-0575-3.pdf> [retrieved on 2024-06-13] | 1-9,16 | TECHNICAL FIELDS SEARCHED (IPC) A61B A61M |
| A | * abstract * <br> * figure 4 * <br> * the whole document * | 10-15,17 | |

-/--

The present search report has been drawn up for all claims

| Place of search | Date of completion of the search | Examiner |
|---|---|---|
| The Hague | 14 June 2024 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

**Application Number**

EP 23 30 7436

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LOISON V ET AL: "Mapping general anesthesia states based on electro-encephalogram transition phases", NEUROIMAGE, [Online] vol. 285, 20 December 2023 (2023-12-20), pages 120498-1, XP093174570, AMSTERDAM, NL ISSN: 1053-8119, DOI: 10.1016/j.neuroimage.2023.120498 Retrieved from the Internet: URL:https://www.sciencedirect.com/science/article/pii/S1053811923006481/pdfft?md5=9c08c0e785db186e6379033c5e9daf45&pid=1-s2.0-S1053811923006481-main.pdf> [retrieved on 2024-06-13] | 1-9,16 | |
| A | * abstract * * the whole document * * figures 2-5 * | 10-15,17 | |
| X | CN 108 325 020 A (UNIV YANSHAN) 27 July 2018 (2018-07-27) | 10-15,17 | |
| A | * abstract * * claims 1-6; figures 1,2 * | 1-9,16 | TECHNICAL FIELDS SEARCHED (IPC) |
| X | WO 2015/067956 A1 (IMP INNOVATIONS LTD [GB]) 14 May 2015 (2015-05-14) | 10-15,17 | |
| A | * abstract * * annex 2, p.71 and further, especially sect 3.4 * * claims 1-8 * | 1-9,16 | |
| X | WO 2023/285387 A1 (BECKER MICHAEL [DE]) 19 January 2023 (2023-01-19) | 10-15,17 | |
| A | * abstract * * page 8, last paragraph - page 9, paragraph first * * page 13, last paragraph * * claims 1-7 * * figure 10b * | 1-9,16 | |

The present search report has been drawn up for all claims

| Place of search | Date of completion of the search | Examiner |
|---|---|---|
| The Hague | 14 June 2024 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.....................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 30 7436

This annex lists the patent family members relating to the patent documents cited in the above-mentioned European search report.
The members are as contained in the European Patent Office EDP file on
The European Patent Office is in no way liable for these particulars which are merely given for the purpose of information.

14-06-2024

| Patent document cited in search report | | Publication date | Patent family member(s) | | Publication date |
|---|---|---|---|---|---|
| CN 108325020 | A | 27-07-2018 | NONE | | |
| WO 2015067956 | A1 | 14-05-2015 | EP | 3066599 A1 | 14-09-2016 |
| | | | US | 2016279329 A1 | 29-09-2016 |
| | | | WO | 2015067956 A1 | 14-05-2015 |
| WO 2023285387 | A1 | 19-01-2023 | DE | 102021117940 A1 | 12-01-2023 |
| | | | EP | 4370015 A1 | 22-05-2024 |
| | | | WO | 2023285387 A1 | 19-01-2023 |

## REFERENCES CITED IN THE DESCRIPTION

**Patent documents cited in the description**

- US 11638529 B

- US 20200390347 A

**Non-patent literature cited in the description**

- **SCHNIDER et al.** *Anesthesiology*, 1998, vol. 88, 1170-82
- **SCHNIDER et al.** *Anesthesiology*, 1999, vol. 90, 1502-16
- **J. CARTAILLER** ; **C. TOUCHARD** ; **P. PARUTTO** ; **E. GAYAT** ; **C. PAQUET** ; **F. VALLÉE**. Brain fragility among middle-aged and elderly patients from electroencephalogram during induction of anaesthesia. *Eur. J. Anaesthesiol. EJA*, December 2021, vol. 38 (12), 1304-1306
- **J. CARTAILLER** ; **P. PARUTTO** ; **C. TOUCHARD** ; **F. VALLÉE** ; **D. HOLCMAN**. Alpha rhythm collapse predicts iso-electric suppressions during anesthesia. *Commun. Biol.*, 2019, vol. 2 (1), 1-10
- **A. R. ABSALOM** ; **K. P. MASON**. Total intravenous anesthesia and target controlled infusions. Springer, 2017
- **D. J. ELEVELD** ; **P. COLIN** ; **A. R. ABSALOM** ; **M. M. R. F. STRUYS**. Pharmacokinetic-pharmacodynamic model for propofol for broad application in anaesthesia and sedation. *Br. J. Anaesth.*, May 2018, vol. 120 (5), 942-959

- **P. L. S. CHAN** ; **P. JACQMIN** ; **M. LAVIELLE** ; **L. MCFADYEN** ; **B. WEATHERLEY**. The use of the SAEM algorithm in MONOLIX software for estimation of population pharmacokinetic-pharmacodynamic-viral dynamics parameters of maraviroc in asymptomatic HIV subjects. *J. Pharmacokinet. Pharmacodyn.*, February 2011, vol. 38 (1), 41-61
- **G. PAPAMAKARIOS** ; **E. NALISNICK** ; **D. J. REZENDE** ; **S. MOHAMED** ; **B. LAKSHMINARAYANAN**. Normalizing flows for probabilistic modeling and inference. *J. Mach. Learn. Res.*, 2021, vol. 22 (1), 2617-2680
- **A. GELMAN** ; **J. B. CARLIN** ; **H. S. STERN** ; **D. B. DUNSON** ; **A. VEHTARI** ; **D. B. RUBIN**. Bayesian Data Analysis. CRC Press, 2013
- **N. KANTAS** ; **A. DOUCET** ; **S. S. SINGH** ; **J. MACIEJOWSKI** ; **N. CHOPIN**. On Particle Methods for Parameter Estimation in State-Space Models. *Stat. Sci.*, August 2015, vol. 30 (3)